# FASCICULE DE BREVET EUROPEEN

(11) **EP 0 922 696 B1**
(45) Date de publication et mention de la délivrance du brevet: **20.04.2005**
(21) Numéro de dépôt: 99103523.9
(22) Date de dépôt: 09.06.1994
(51) Int. Cl.: C07D 209/20, C07C 323/59, C07D 243/04, C07D 239/36, C07K 5/097

(54) **Uréines dérivées d'alpha, oméga-diaminoacides et peptides**
Harnstoffderivate von alpha, omega-Diaminosäuren und peptiden
Urea derivatives of alpha, omega-diaminoacids and peptides

(30) Priorité: 18.06.1993 BE 9300621
(43) Date de publication de la demande: 16.06.1999
(62) Demande divisionnaire de: 94201643.7
(73) Titulaire: SOLVAY (Société Anonyme), 1050 Bruxelles (BE)
(72) Inventeur: Callens, Roland, 9031 Drongen (BE); Blondeel, Georges, 9300 Aalst (BE); Anteunis, Marc, 9030 Mariakerke (BE); Becu, Frank, 8490 Jabbeke (BE)
(74) Mandataire: Jacques, Philippe

(56) Documents cités:
- DE-A- 2 449 167
- US-A- 2 489 233
- US-A- 2 650 921
- US-A- 2 773 872
- M. SUZUKI, ET AL.: "Practical synthesis of optically pure 1- and 3-substituted dihydroorotic acids" CHEMICAL AND PHARMACEUTICAL BULLETIN, vol. 37, no. 7, juillet 1989 (1989-07), pages 1764-1769, XP002222187 Pharmaceutical Society of Japan, Tokyo, JP ISSN: 0009-2363
- J.T. CAPECCHI, ET AL.: "Critical examination of a method for the analysis of alpha and omega linkages in peptides containing aspartic acid and glutamic acid" JOURNAL OF ORGANIC CHEMISTRY, vol. 48, no. 12, 17 juin 1983 (1983-06-17), pages 2014-2021, XP000574968 American Chemical Society, Washington, DC, US ISSN: 0022-3263
- A.R. KATRITZKY, ET AL.: "beta-Ureido acids and dihydrouracils - VII. Applications of proton resonance spectroscopy - XXXII. NMR spectra and conformation of dihydrouracils and related compounds" TETRAHEDRON, vol. 25, no. 17, septembre 1969 (1969-09), pages 3807-3824, XP002222188 Elsevier Science Publishers, Amsterdam, NL ISSN: 0040-4020
- M. EGLI, ET AL.: "Synthese von racemischen Aminozuckersäure-lactonen: xylo- und lyxo-2,3-Diacetylamino-5- acetoxypentan-4-olid und -2,3,5-Triacetylaminopentan-4-olid" HELVETICA CHIMICA ACTA, vol. 69, no. 6, 10 septembre 1986 (1986-09-10), pages 1442-1460, XP002222189 Verlag Helvetica Chimica Acta, Basel, CH ISSN: 0018-019X
- J.L. ADAMS, ET AL.: "cis-4-Carboxy-6- (mercaptomethyl)-3,4,5,6-tetrahydro- pyrimidin-2(1H)-one, a potent inhibitor of mammalian dihydroorotase" JOURNAL OF MEDICINAL CHEMISTRY, vol. 31, no. 7, juillet 1988 (1988-07), pages 1355-1359, XP001074178 American Chemical Society, Washington, DC, US ISSN: 0022-2623
- M. SUZUKI, ET AL.: "Synthesis and central nervous system actions of thyrotropin- releasing hormone analogues containing a dihydroorotic acid moiety" JOURNAL OF MEDICINAL CHEMISTRY, vol. 33, no. 8, août 1990 (1990-08), pages 2130-2137, XP002186256 American Chemical Society, Washington, DC, US ISSN: 0022-2623
- J.T. CAPECCHI, ET AL.: "Substrate specificity of pyroglutamylaminopeptidase" JOURNAL OF MEDICINAL CHEMISTRY, vol. 28, no. 1, janvier 1985 (1985-01), pages 140-143, XP002222190 American Chemical Society, Washington, DC, US ISSN: 0022-2623
- T. SZIRTES, ET AL.: "Synthesis of thyrotropin-releasing hormone analogues. 2. Tripeptides structurally greatly differing from TRH with high central nervous system activity" JOURNAL OF MEDICINAL CHEMISTRY, vol. 29, no. 9, septembre 1986 (1986-09), pages 1654-1658, XP002222191 American Chemical Society, Washington, DC, US ISSN: 0022-2623

## Description

La présente invention se rapporte à de nouvelles uréines, dérivées d'α,ω-diaminoacides.

Suzuki, J. Med. Chem, 1990, 33, 2130-37, DE-0S-2449167 and Szirtes, J. Med. Chem, 1986, 22, 1654-58 décrivent des acides 2-oxo-imidazolidine-4-carboxyliques et des peptides dérivés.

L'invention concerne des N^{ω}-(aminoacidocarbonyl)-α,ω-diaminoacides, uréines dérivées d'un α,ω-diaminoacide, de formule générale 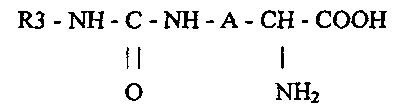 dans laquelle A représente un groupement bivalent, constitué d'une chaîne carbonée linéaire formée de 4 à 8 atomes de carbone, laquelle est éventuellement substituée par un ou plusieurs groupements choisis parmi les groupements alkyles en C1-C3 et les groupements fonctionnels comprenant au moins un atome d'oxygène ou de soufre tels qu'un groupement carboxyle, acyle, hydroxyle, alcoxyle ou mercapto et dans laquelle R3 - NH représente un aminoacide ou un peptide. De préférence A est un groupement polyméthylène contenant 4 ou 5 atomes de carbone. R3 - NH est de préférence un aminoacide et, plus préférentiellement, un aminoacide essentiel.

Ces composés nouveaux constituent des composés de structure proche de celle de dipeptides et sont utilisables notamment en lieu et place des dipeptides correspondants, notamment comme source d'aminoacides essentiels en alimentation humaine parentérale ou en alimentation animale.

L'invention concerne également les uréines cycliques de formule générale 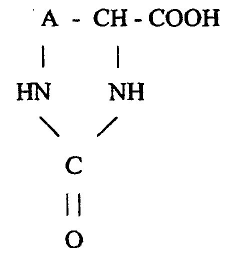 dans laquelle A représente un groupement bivalent, constitué d'une chaîne carbonée linéaire formée de 3 atomes de carbone, laquelle est éventuellement substituée par un ou plusieurs groupements choisis parmi les groupements alkyles en C1-C3 et les groupements fonctionnels comprenant au moins un atome d'oxygène ou de soufre tels qu'un groupement carboxyle, acyle, hydroxyle, alcoxyle ou mercapto. De manière particulièrement préférée, A représente un groupement triméthylène -(CH₂)₃-. Dans ce cas, l'uréine formée est l'acide 2-oxo-hexahydro-1,3-diazépine-4-carboxylique.

Les uréines selon l'invention peuvent être obtenus selon le procédé décrit dans la demande initiale EP-A-0629612.

Selon que l'énantiomère (D) ou (L) du diaminoacide est mis en oeuvre dans le procédé de fabrication, l'énantiomère (D) ou (L) de l'uréine cyclique correspondante est obtenu sous forme chiralement pure.

Ces uréines cycliques peuvent être utilisées notamment comme résidu N-terminal de certains peptides biologiquement actifs, tels l'hormone TRH (Thyrotropin Releasing Hormone), en remplacement du groupement pyroglutamyl N-terminal de ce peptide. L'invention concerne enfin des peptides analogues de la TRH, de formule générale 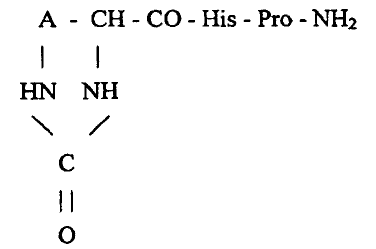 dans laquelle A est un groupement bivalent, constitué d'une chaîne carbonée linéaire formée de 3 atomes de carbone, laquelle est éventuellement substituée par un ou plusieurs groupements choisis parmi les groupements alkyles en C1-C3 et les groupements fonctionnels comprenant au moins un atome d'oxygène ou de soufre tels qu'un groupement carboxyle, acyle, hydroxyle, alcoxyle ou mercapto. De préférence, A est un groupement triméthylène. Ces peptides présentent une résistance accrue à la digestion protéolytique, tout en conservant une activité biologique élevée.

Par uréine, on entend désigner tout composé dont la structure moléculaire contient la structure -NH-CO-NH-.

Par aminoacide, on entend désigner, aux fins de la présente invention, tout composé comprenant au moins un groupement amino et au moins un groupement carboxyle. Par extension, on entend aussi englober ci-après sous le vocable "aminoacide", tout aminoacide dont certains autres groupements sont éventuellement liés à des groupements organiques tels que des groupements protecteurs. En particulier, par α,ω-diaminoacide, on entend désigner tout aminoacide comprenant au moins un groupement amino et au moins un groupement carboxyle liés au même atome de carbone de la molécule et comprenant en outre au moins un autre groupement amino lié à un autre atome de carbone. Il s'agit le plus souvent d'un composé de formule générale 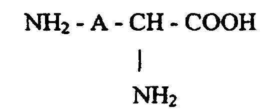 dans laquelle A représente un groupement bivalent, constitué d'une chaîne carbonée de 1 à 8 atomes de carbone, laquelle est éventuellement substituée par un ou plusieurs groupements choisis parmi les groupements alkyles en C1-C3 et les groupements fonctionnels comprenant au moins un atome d'oxygène ou de soufre tels qu'un groupement carboxyle, acyle, hydroxyle, alcoxyle ou mercapto, sans que le nombre total d'atomes de carbone dans le groupement A ne soit supérieur à 15. De préférence, A est un groupement polyméthylène comprenant de 2 à 5 atomes de carbone. A titre d'exemples d'α,ω-diaminoacides, on peut citer notamment l'acide 2,3-diaminopropanoïque, l'acide 2,4-diaminobutanoïque, l'ornithine, la lysine, l'homolysine, la 5-hydroxylysine, la 6-méthyllysine et l'acide 2,6-diaminopimélique.

Les représentations symboliques des aminoacides et des peptides adoptées dans la description et les exemples suivent les recommandations IUPAC de nomenclature, généralement adoptées et décrites par exemple dans "Nomenclature and Symbolism for Amino Acids and Peptides, Recommendations 1983", Eur. J. Biochem. (1984), 138, p. 9-37. Sauf stipulation contraire, tous les aminoacides décrits sont les (L)-aminoacides.

Les exemples suivants illustrent l'invention.

Les différents produits et intermédiaires de synthèse rapportés dans les exemples ont été caractérisés par différentes méthodes analytiques, mises en oeuvre dans les conditions suivantes :
- rotation optique (α) : mesurée à 589 nm à 25 °C
- Chromatographie en couche mince (CCM) :
   . Plaques de silicagel MERCK 60F-254
   . éluants :
      Rf(1) Acétate d'éthyle:n-butanol:acide acétique:eau 1:1:1:1
      Rf(2) Acétonitrile:chloroforme:acide acétique:eau 5:2:2:1
      Rf(3) Acétonitrile:chloroforme:acide acétique:eau 7:4:4:2
- Chromatographie HPLC :
   . Colonne C-18 Vydac 5µm
   . Elution : gradient de 98 % A + 2 % B jusqu'à 25 % A + 75 % B en 49 minutes (A = eau 0,1 % en acide trifluoroacétique ; B = acétonitrile 0,1 % en acide trifluoroacétique)
   . Débit = 2 ml/min
   . Détection : UV 220 nm.
- Résonance magnétique nucléaire (RMN) :
   . Appareil Brüker AMX 500 MHz
   . Shift donnés en ppm
   . Allures des résonances : m=multiplet, s=singulet, d=doublet, t=triplet, q=quadruplet, quint=quintuplet, o=octuplet.

### Exemple 1 : Synthèse de N^{ε}-(N^{α}-tryptophanocarbonyl)-lysine

5,1 g (25 mmol) de tryptophane, 1,34 g (5 mmol) de N^{ε}-phényloxycarbonyl-lysine et 1,05 g (25 mmol) de LiOH.H₂O ont été pesés dans un ballon de 100 ml, puis 40 ml d'eau ont été introduits dans le ballon. Celui-ci a été immergé dans un bain d'huile maintenu à 75°C pendant 45 minutes, a ensuite été rapidement refroidi à température ambiante sous de l'eau courante, puis traité avec 25 ml d'acide chlorhydrique. Le précipité formé a été filtré, après une nuit au réfrigérateur.

Une analyse HPLC a révélé une conversion complète de la N^{ε}-phényloxycarbonyl-lysine en 2 produits présentant, dans les conditions d'analyse, un temps de rétention (tR) de 12,23 et 13,95 minutes, avec un rapport 13 : 1 en surface de pics. Les produits ont été séparés par injection du filtrat tel quel sur une colonne HPLC C-18 préparative, puis lyophilisés. 880 mg de N^{ε}-(N^{α}-tryptophanocarbonyl)-lysine et 76 mg de N^{ε}-(N^{ε}-(N^{α}-tryptophanocarbonyl)-N^{α}-lysinocarbonyl)-lysine ont été obtenus.
Les propriétés physico-chimiques de la N^{ε}-(N^{α}-tryptophanocarbonyl)-lysine sont les suivantes :
PF : 133°C
α : + 3,31 (c = 1, 1 % acide acétique)
CCM : Rf(3) = 0,34
RMN (H-1) dans le DMSO-d6 :

| | |
|---|---|
| 11,00 (1H s) NH indole | 7,50 (1H d) H4 indole |
| 7,32 (1H d) H7 indole | 7,09 (1H s) H2 indole |
| 7,03 (1H t) H6 indole | 6,94 (1H t) H5 indole |
| 6,28 (1H t large) εNH Lys | 6,18 (1H d) αNH Trp |
| 4,36 (1H m) Hα Trp | 3,30 (1H m) Hα Lys |
| 3,12 (1H dd) HβATrp | 2,99 (1H dd) HβBTrp |
| 2,94 (2H m) Hε's Lys | 1,70 (1H m) HβA Lys |
| 1,60 (1H m) HβB Lys | 1,31 (4H m) Hγ's + Hδ's Lys |

### Exemple 2 : Synthèse de N^{ε}-(méthioninocarbonyl)-lysine

La N^{ε}-(méthioninocarbonyl)-lysine a été préparée au départ de méthionine et de N^{ε}-phényloxycarbonyl-lysine selon la même recette que celle décrite à l'exemple 1. Elle présente les propriétés physico-chimiques suivantes :
PF : 148°C
α : - 9,5 (c = 1, 1 % acide acétique)
CCM : Rf(3) = 0,27
HPLC : tR = 7,14 min
RMN (H-1) dans le DMSO-d6 :

| | |
|---|---|
| 6,43 (1H d large) NHα Met | 6,33 (1H t large) NHε Lys . |
| 4,11 (1H m) Hα Met | 3,35 (1H m) Hα Lys |
| 2,95 (2H m) Hε's Lys | 2,33 (2H t) Hγ's Met |
| 2,02 (3H s) CH3 Met | 1,88 (1H m) HβA Met |
| 1,77 (1H m) HβB Met | 1,72 (1H m) HβA Lys |
| 1,65 (1H m) HβB Lys | 1,33 (4H m) Hγ's + Hδ's Lys |

### Exemple 3 : synthèse de l'acide (D)-2-oxo-hexahydro-1,3-diazépine-4-carboxylique

A une suspension de 1,27 g (5 mmol) de (D)-N^{δ}-phényloxycarbonyl-ornithine dans 15 ml de diméthoxyéthane et 10 ml d'eau ont été ajoutés 7,7 ml (± 100 mmol) d'ammoniaque à 25 %. Le degré de conversion a été suivi par CCM. Une fois la (D)-phényloxycarbonyl-ornithine disparue, le milieu réactionnel a été concentré à sec. Le résidu a été trituré par 20 ml d'acétone à 80 %, filtré et séché. A ce stade, on a déterminé par RMN que le produit brut obtenu était le sel d'ammonium de l'acide (D)-2-oxo-hexahydro-1,3-diazépine-4-carboxylique, contaminé par environ 5 % de citrulline (Rendement : 820 mg ou 92 % en produit brut). L'acide (D)-2-oxo-hexahydro-1,3-diazépine-4-carboxylique a été obtenu, avec une pureté supérieure à 98 %, par passage du produit brut en solution aqueuse à travers une colonne résine échangeuse d'ions sous forme H⁺, puis lyophilisation.
PF : 130-150 °C (décomposition)
α : + 14,6 (c = 1, eau)
CCM : Rf(3) : 0,70 ne réagit plus à la ninhydrine
Rf(3) : 0,16 pour la citrulline
Rf(3) : 0,54 pour la N^{δ}-phényloxycarbonyl-ornithine
RMN(H) réf DMSO-d6 à 2,49 :

| | |
|---|---|
| 6,20 (1H,s large) NH1 | 5,55 (1H,s large) NH3 |
| 3,75 (1H,m) H4 | 2,89 (2H,m) H7 |
| 1,90 (1H,m) H5A | 1,77 (1H,m) H5B |
| 1,55 (2H,m) H's 6 | |

RMN(C-13) réf DMSO-d6 à 39,50 :

| | |
|---|---|
| 173,45 (COOH) | 163,65 (C2) |
| 54,43 (C4) | 31,03 (C5) |
| 26,81 (C6) | |

### Exemple 4 : Synthèse de (2-oxo-hexahydro-1,3-diazépine-4-carbonyl)-His-Pro-NH₂

1 g (1,40 mmol) de bis-trifluoroacétate de N^{δ}(phényloxycarbonyl)Orn-His-Pro-NH₂ a été dissous dans 10 ml de méthanol contenant O,75 ml (5,4 mmol) de triéthylamine. La solution a été portée à léger reflux à une température d'environ 65 °C, jusqu'à disparition du produit de départ (contrôle par CCM). La solution a été concentrée à sec et triturée avec 20 ml de dichlorométhane. Après filtration, le produit brut recueilli (0,52 g) a été purifié par chromatographie préparative sur phase inverse C-4. L'échantillon analytique isolé sous forme de sel d'acétate présente les propriétés physico-chimiques suivantes :
α = -8,1 (c = 1, 1 % acide acétique)
PF : 105 °C
CCM Rf(1) = 0,42
RMN (¹H, D20) : certaines résonances sont dédoublées à cause de l'isomerie cis/trans au niveau de la liaison His-Pro. Les résultats pour la forme majeure (± 85 %) sont repris ci-après :

| | |
|---|---|
| 8,56 (1H s) H2 imidazole | 7,34 (1H s) H5 imidazole |
| 5,08 (1H dd) Hα His | 4,46 (1H dd) Hα Pro |
| 4,07 (1H dd) Hα Odc | 3,81 (1H m) HδA Pro |
| 3,65 (1H m) HδB Pro | 3,30 (1H dd) HβA His |
| 3,19 (1H dd) HβB His | 3,08 à 3,02 (2H m) HδA+B Odc |
| 2,36 (1H m) HβA Pro | 2,15 à 1,95 (5H + CH3 acétate) |
| 1,74 (1H m) HγA Odc | 1,51 (1H m) HγB Odc |

## Revendications

1. Uréines cycliques de formule générale 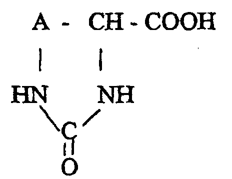 dans laquelle A représente un groupement bivalent, constitué d'une chaîne carbonée linéaire formée de 3 atomes de carbone, laquelle est éventuellement substituée par un ou plusieurs groupements choisis parmi les groupements alkyles en C1-C3 et les groupements fonctionnels comprenant au moins un atome d'oxygène ou de soufre tels qu'un groupement carboxyle, acyle, hydroxyle, alcoxyle ou mercapto.

2. Uréine selon la revendication 1 dans laquelle A représente un groupement triméthylène -(CH₂)₃-.

3. Utilisation d'une uréine cyclique selon la revendication 1 ou 2 comme résidu N-terminal de peptides.

4. Peptides de formule générale 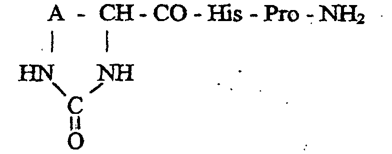 dans laquelle A est un groupement bivalent, constitué d'une chaîne carbonée linéaire formée de 3 atomes de carbone, laquelle est éventuellement substituée par un ou plusieurs groupements choisis parmi les groupements alkyles en C1-C3 et les groupements fonctionnels comprenant au moins un atome d'oxygène ou de soufre tels qu'un groupement carboxyle, acyle, hydroxyle, alcoxyle ou mercapto.

5. Peptide selon la revendication 4 dans lequel A représente un groupement triméthylène -(CH₂)₃-.

6. N^{ω}-(aminoacidocarbonyl)-α,ω-diaminoacides de formule générale 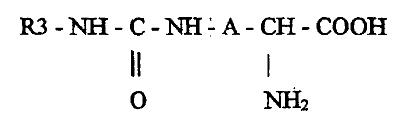 dans laquelle A représente un groupement bivalent, constitué d'une chaîne carbonée linéaire formée de 4 à 8 atomes de carbone, laquelle est éventuellement substituée par un ou plusieurs groupements choisis parmi les groupements alkyles en C1-C3 et les groupements fonctionnels comprenant au moins un atome d'oxygène ou de soufre tels qu'un groupement carboxyle, acyle, hydroxyle, alcoxyle ou mercapto et dans laquelle R3 - NH représente un aminoacide ou un peptide.

7. N^{ω}-(aminoacidocarbonyl)-α,ω-diaminoacides selon la revendication 6, dans lesquels A est un groupement polyméthylène contenant 4 ou 5 atomes de carbone.

8. N^{ω}-(aminoacidocarbonyl)-α,ω-diaminoacides selon la revendication 6 ou 7, dans lesquels R3 - NH est un aminoacide.

9. Utilisation de N^{ω}-(aminoacidocarbonyl)-α,ω-diaminoacides selon l'une quelconque des revendications 6 à 8, comme source d'aminoacides essentiels en alimentation humaine parentérale ou en alimentation animale.

## Patentansprüche

1. Cyclische Harnstoffe mit der allgemeinen Formel 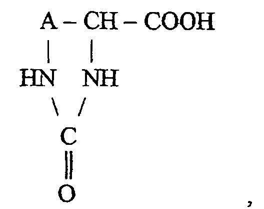 worin A eine zweiwertige Gruppe darstellt, die aus einer geraden, aus 3 Kohlenstoffatomen gebildeten Kohlenstoffkette besteht, die gegebenenfalls durch eine oder durch mehrere Gruppen substituiert ist, ausgewählt unter den C₁-C₃-Alkylgruppen und den funktionellen Gruppen, die wenigstens ein Sauerstoff- oder Schwefelatom enthalten, wie einer Carboxy-, Acyl-, Hydroxy-, Alkoxy- oder Mercaptogruppe.

2. Harnstoff nach Anspruch 1, worin A eine Trimethylengruppe -(CH₂)₃- darstellt.

3. Verwendung eines cyclischen Harnstoffes nach Anspruch 1 oder 2 als N-endständiger Peptidrest.

4. Peptide mit der allgemeinen Formel 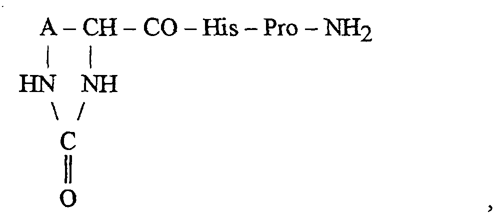 worin A eine zweiwertige Gruppe darstellt, die aus einer geraden, aus 3 Kohlenstoffatomen gebildeten Kohlenstoffkette besteht, die gegebenenfalls durch eine oder durch mehrere Gruppen substituiert ist, ausgewählt unter den C₁-C₃-Alkylgruppen und den funktionellen Gruppen, die wenigstens ein Sauerstoff- oder Schwefelatom enthalten, wie einer Carboxy-, Acyl-, Hydroxy-, Alkoxy- oder Mercaptogruppe.

5. Peptid nach Anspruch 4, worin A Trimethylengruppe -(CH₂)₃- darstellt.

6. N^{ω}-(Aminoacidocarbonyl)-α,ω-diaminosäuren mit der allgemeinen Formel 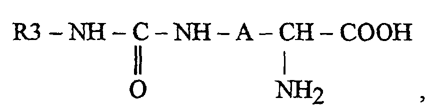 worin A eine zweiwertige Gruppe darstellt, die aus einer geraden, aus 4-8 Kohlenstoffatomen gebildeten Kohlenstoffkette besteht, die gegebenenfalls durch eine oder durch mehrere Gruppen substituiert ist, ausgewählt unter den C₁-C₃-Alkylgruppen und den funktionellen Gruppen, die wenigstens ein Sauerstoff- oder Schwefelatom enthalten, wie einer Carboxy-, Acyl-, Hydroxy-, Alkoxy- oder Mercaptogruppe, und worin R3-NH eine Aminosäure oder ein Peptid darstellt.

7. N^{ω}-(Aminoacidocarbonyl)-α,ω-diaminosäuren nach Anspruch 6, worin A eine 4 oder 5 Kohlenstoffatome enthaltende Polymethylengruppe bedeutet.

8. N^{ω}-(Aminoacidocarbonyl)-α,ω-diaminosäuren nach Anspruch 6 oder 7, worin R3-NH eine Aminosäure darstellt.

9. Verwendung von N^{ω}-(Aminoacidocarbonyl)-α,ω-diaminosäure nach einem der Ansprüche 6 bis 8 als Quelle für essentielle Aminosäuren in der parenteralen menschlichen Ernährung oder in der tierischen Ernährung.

## Claims

1. Cyclic urea derivatives of general formula 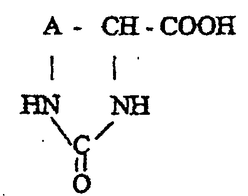 in which A represents a divalent group composed of a linear carbon chain formed of 3 carbon atoms which is optionally substituted by one or more groups chosen from C₁-C₃ alkyl groups and functional groups comprising at least one oxygen or sulphur atom, such as a carboxyl, acyl, hydroxyl, alkoxy or mercapto group.

2. Urea derivative according to Claim 1, in which A represents a trimethylene group -(CH₂)₃-.

3. Use of a cyclic urea derivative according to Claim 1 or 2 as N-terminal residue of peptides.

4. Peptides of general formula 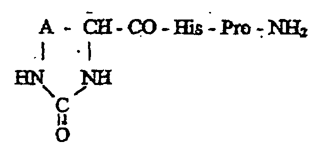 in which A is a divalent group composed of a linear carbon chain formed of 3 carbon atoms which is optionally substituted by one or more groups chosen from C₁-C₃ alkyl groups and functional groups comprising at least one oxygen or sulphur atom, such as a carboxyl, acyl, hydroxyl, alkoxy or mercapto group.

5. Peptide according to Claim 4, in which A represents a trimethylene group -(CH₂)₃-.

6. N^{ω}-(Aminoacidocarbonyl)-α,ω-diamino acids of general formula 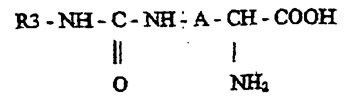 in which A represents a divalent group composed of a linear carbon chain formed of 4 to 8 carbon atoms which is optionally substituted by one or more groups chosen from C₁-C₃ alkyl groups and functional groups comprising at least one oxygen or sulphur atom, such as a carboxyl, acyl, hydroxyl, alkoxy or mercapto group, and in which R3-NH represents an amino acid or a peptide.

7. N^{ω}-(Aminoacidocarbonyl)-α,ω-diamino acids according to Claim 6, in which A is a polymethylene group comprising 4 or 5 carbon atoms.

8. N^{ω}-(Aminoacidocarbonyl)-α,ω-diamino acids according to Claim 6 or 7, in which R3-NH is an amino acid.

9. Use of N^{ω}-(aminoacidocarbonyl)-α,ω-diamino acids according to any one of Claims 6 to 8 as source of essential amino acids in human parenteral nutrition or in animal nutrition.
